(19) Europäisches Patentamt
European Patent Office
Office européen des brevets

(11) Veröffentlichungsnummer : **0 064 635**
**B1**

(12) # EUROPÄISCHE PATENTSCHRIFT

(45) Veröffentlichungstag der Patentschrift : **21.11.85**

(51) Int. Cl.⁴ : **C 07 H 15/20**, A 61 K 31/70

(21) Anmeldenummer : **82103388.3**

(22) Anmeldetag : **22.04.82**

(54) **Aminocyclitderivate, ihre Herstellung und sie enthaltende Arzneimittel.**

(30) Priorität : **05.05.81 DE 3117705**
**19.08.81 DE 3132691**

(43) Veröffentlichungstag der Anmeldung : **17.11.82 Patentblatt 82/46**

(45) Bekanntmachung des Hinweises auf die Patenterteilung : **21.11.85 Patentblatt 85/47**

(84) Benannte Vertragsstaaten : **AT BE CH DE FR GB IT LI NL SE**

(56) Entgegenhaltungen :
**FR-A- 2 375 248**
**THE JOURNAL OF ANTIBIOTICS, Band XXXIV, Nr. 11, November 1981, Seiten 1429-1433, Japan Antibiotics Research Association, Tokyo, JP. S. OMOTO et al.: "Oligostatins, new antibiotics with amylase inhibitory activity"**
**"Advances in Carbohydrate Chemistry" Band 18, S. 268-73 und 292-5, Band 23, S. 122-9 und 152-5**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Heiker, Fred Robert, Dr.**
**Pahlkestrasse 15**
**D-5600 Wuppertal 1 (DE)**
Erfinder : **Müller, Lutz, Dr.**
**Kronprinzenallee 111**
**D-5600 Wuppertal 1 (DE)**
Erfinder : **Puls, Walter, Dr.**
**In den Birken 75**
**D-5600 Wuppertal 1 (DE)**
Erfinder : **Bischoff, Hilmar, Dr.**
**Wilkhausstrasse 107**
**D-5600 Wuppertal 2 (DE)**

**EP 0 064 635 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Aminocyclitderivate und ein Verfahren zu ihrer Herstellung. Die erfindungsgemäßen Verbindungen lassen sich als Arzneimittel verwenden, insbesondere in der Therapie von Diabetes, Adipositas und Hyperlipämie.

Die erfindungsgemäßen Verbindungen haben die allgemeine Formel I

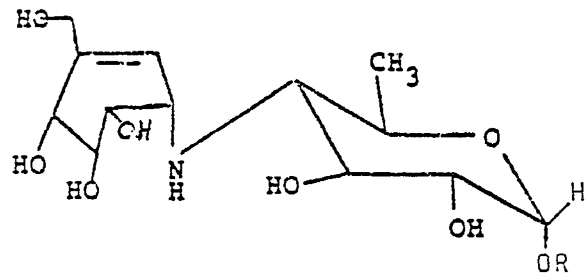

(I)

(α-Anomeres)

worin R einen gegebenenfalls durch Alkoxy mit 1 bis 4 Kohlenstoffatomen, Alkylthio mit 1 bis 4 Kohlenstoffatomen, Halogen, oder Cyan, ein- oder mehrfach substituierten Alkyl- oder Alkenylrest mit bis zu 6 C-Atomen darstellt.

Die Verbindungen der Formel I sind kristallin.

Aus der DE-OS 2 726 207 sind Verbindungen der Formel II

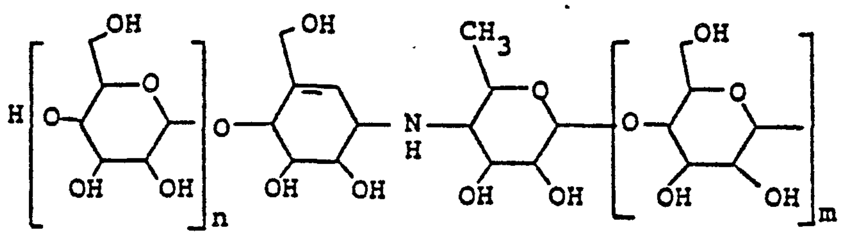

(II)

bekannt. Sie können erhalten werden durch Umsetzung der entsprechenden Acetobromverbindungen mit den jeweiligen Alkoholen. Allerdings ist dieses Verfahren zur Darstellung von Verbindungen II mit $n + m = 0$ insofern noch nicht optimal, als die Synthese in diesem speziellen Fall aufwendig ist und vergleichsweise geringe Ausbeuten ergibt.

Die vorliegende Erfindung stellt nun Verbindungen der allgemeinen Formel I bereit.

Für R in der Bedeutung von Alkyl seien beispielhaft Methyl, Ethyl, n-Propyl, i-Propyl, n-Butyl, t-Butyl und n-Hexyl genannt, besonders bevorzugt sind Methyl, Ethyl und i-Propyl.

Beispielhaft für einen substituierten Alkylrest sei hier $—CH_2—CH_2—Cl$ genannt.

R in der Bedeutung von Alkenyl steht für geradkettige oder verzweigte Alkenylreste mit 1-3 Doppelbindungen (z. B. Allyl).

Es wurde überraschend gefunden, daß man die erfindungsgemäßen Verbindungen der Formel I erhält, wenn Substanzen der Formel III

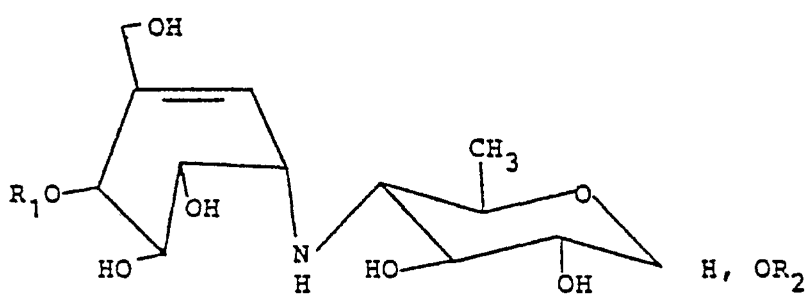

(III)

worin $R_1$ und $R_2$ für gleiche oder verschiedene Mono- oder Oligosaccharidreste stehen, die als $R_1$ glykosidisch und als $R_2$ beliebig über Sauerstoff an das Restmolekül gebunden sind und wobei die Verbindungen der Formel III gegebenenfalls auch in Form ihrer 0-geschützten Derivate, vorliegen können,

mit einem Alkohol ROH in Gegenwart von Protonensäuren HX umgesetzt werden, worin R die oben genannte Bedeutung hat und X den Rest einer starken anorganischen nicht oxidierenden Säure darstellt und das α-Anomere aus dem anfallenden Anomerengemisch isoliert.

In der Formel III soll die Schreibweise H, $OR_2$ zum Ausdruck bringen, daß beide möglichen stereoisomeren Verbindungen umfaßt sind.

Die erwähnten Schutzgruppen sind die in der Zuckerchemie üblichen, beispielsweise die Acetylgruppe.

Das Verfahren, Oligosaccharide durch wäßrige Protonensäuren zu spalten, ist bekannt. Setzt man jedoch Verbindungen der Formel III in diese Reaktion ein, so gelangt man nicht zu der Verbindung I mit R = H, sondern erhält das tricyclische Oxazolidin IV.

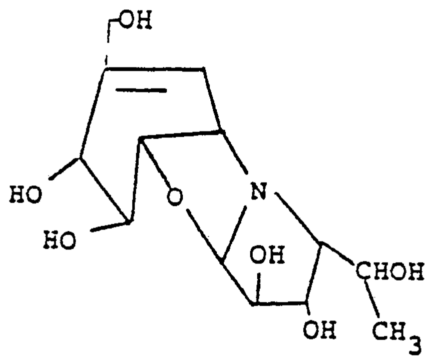

(IV)

Mit diesem Ergebnis mußte sogar gerechnet werden, denn umfangreiche Arbeiten von Paulsen und Mitarbeitern haben gezeigt, daß unter sauren Bedingungen bei Kohlenhydraten mit Amino- und Alkylaminogruppen in 4-Stellung besonders leicht eine Kondensation dieser nucleophilen Gruppierung mit der Aldehydfunktion unter Bildung von Piperidin und Pyrrolidin-Zuckern eintritt. Diese Ringbildung und die sich anschließenden Folgereaktionen (Eliminierung, Polymerisation, Aromatisierung) werden für eine intensive Zersetzung der Substanzen verantwortlich gemacht und so gibt es in der Literatur zahlreiche Beispiele dafür, daß beim Versuch der Freisetzung von 4-Aminozuckern durch saure Hydrolyse ihrer Derivate, z. B. Glykosiden, nur eine tiefgreifende Zersetzung und teilweise Abscheidung schwarzer polymerer Substanzen beobachtet wird.

Nach diesen Literaturbefunden ist es um so überraschender, daß bei der erfindungsgemäßen Umsetzung die Verbindungen der Formel I in guten Ausbeuten zunächst als Anomerengemisch als kristalline Substanzen erhältlich sind.

Das Verfahren ist dann besonders vorteilhaft, wenn man als Ausgangsverbindungen Substanzen der Formel III wählt, in der $R_1$ und $R_2$ Glucose oder aus Glucose aufgebaute Oligosaccharidreste sind.

Im speziellen wird das erfindungsgemäße Verfahren wie folgt durchgeführt:

Die Verbindungen der Formel I im Gemisch mit den entsprechenden β-Anomeren werden durch Umsetzung der Ausgangsverbindungen III mit dem Alkohol ROH, in Anwesenheit der anorganischen Säure, bei Temperaturen von 60 bis 130 °C vorzugsweise 60-100 °C während mindestens 0,5 Stunden, im allgemeinen 0,5 bis 12 Stunden, erhalten. Als anorganische Säuren werden nicht oxidierende starke Säuren, z. B. Schwefelsäure, Phosphorsäure, vorzugsweise Chlorwasserstoffsäure eingesetzt.

Bei der Durchführung des erfindungsgemäßen Verfahrens sollte besonders folgendes beachtet werden:

Die Säurekonzentration soll im allgemeinen 0,5 bis 4 normal sein. Allerdings ist die Konzentration in diesem Bereich nicht beliebig, sondern in Abhängigkeit vom verwendeten Alkohol zu wählen. Hierbei muß die jeweils im Einzelfall zu wählende optimale Konzentration gegebenenfalls durch einige Vorversuche ermittelt werden. Soviel aber kann gesagt werden, daß im Falle von Methanol die Säurekonzentration im oberen Bereich bewegen kann, ohne, daß es zu wesentlichen Zersetzungen kommt. Geht man jedoch auf Ethanol über, so sollte die Säurekonzentration 1-2 N nicht überschreiten.

Auch hinsichtlich der Reaktionstemperatur ist das Verfahrensoptimum in Abhängigkeit von dem Alkohol und der Säurestärke zu ermitteln.

Allgemein führen zu milde Bedingungen nur zu einer partiellen Spaltung der eingesetzten Verbindungen III und unter zu drastischen Bedingungen kommt es zur überwiegenden Bildung der Substanz IV bzw. zu den erwähnten Zersetzungsreaktionen.

Nach Beendigung der Umsetzung wird die erhaltene Reaktionsmischung mit $Na_2CO_3$ neutralisiert, zur Abtrennung fester Bestandteile filtriert und bei vermindertem Druck konzentriert. Die verbleibende, viskose Masse wird in Wasser aufgenommen und zur Abtrennung nichtbasischer Reaktionsprodukte über einen sauren Ionenaustauscher chromatographiert. Die Eluation der Substanzen der Formel I (im Gemisch mit ihren β-Anomeren) erfolgt mit etwa 1 %iger wäßriger Ammoniaklösung.

Zur Abtrennung noch verbliebener basischer Bestandteile wird das basische Eluat eingeengt und über Aluminiumoxid mit Methanol/Wasser 3 : 1 (V/V) chromatographiert. Die substanzhaligen Eluate werden konzentriert und die von den Nebenprodukten befreiten Verbindungen aus Methanol oder einer Lösungsmittelmischung aus Methanol und Isopropanol umkristallisiert. Das erhaltene Kristallisat bildet

ein Konglomerat aus Kristallen der α- und β-Glykoside der Verbindungen I. Durch fraktionierte Kristallisation und gegebenenfalls selektive Schutzgruppenchemie können dann die reinen Anomere erhalten werden. Im allgemeinen sind die β-Derivate in Methanol weniger löslich, als die α-Derivate und können so durch Umkristallisieren rein erhalten werden. Zur Reindarstellung der α-Derivate empfiehlt sich eine Aufarbeitung unter Einführung von Schutzgruppen.

Die erfindungsgemäßen Verbindungen sind Inhibitoren von Glykosidhydrolasen und eignen sich daher zur Behandlung von Krankheiten, bei denen eine Inhibition dieser Enzyme wünschenswert erscheint. Die Aktivität der Substanzen der Formel I wurde im Saccharase-Inhibitionstest in vitro bestimmt.

Saccharase-Inhibitionstest in vitro

Der Saccharase-Inhibitionstest in vitro ermöglicht die Bestimmung der enzyminhibitorischen Aktivität einer Substanz durch den Vergleich der Aktivität des solubilisierten intestinalen Disaccharidasen-Komplexes in Gegenwart bzw. in Abwesenheit (sog. 100 %-Wert) des Inhibitors. Als Substrat, welches die Spezifität des Inhibitionstestes bestimmt, dient dabei eine praktisch Glucose-freie Saccharose (Glucose < 100 ppm); die Enzymaktivitätsbestimmung basiert auf der spektrophotometrischen Bestimmung freigesetzter Glucose mittels Glucose-Dehydrogenase und Nicotinamid-adenin-dinucleotid als Cofaktor.

Eine Saccharase-Inhibitor-Einheit (SIE) ist definiert als dienenige inhibitorische Aktivität, welche in einem definierten Testansatz eine vorgegebene saccharolytische Aktivität um eine Einheit (Saccharase-Einheit = SE) reduziert; die Saccharase-Einheit ist dabei als diejenige Enzymaktivität definiert, welche untervorgegebenen Bedingungen ein μmol Saccharose pro min spaltet und damit zur Freisetzung von je ein μmol Glucose, welche im Test bestimmt wird, und Fructose, welche im Test nicht erfaßt wird, führt.

Der intestinale Disaccharidasen-Komplex wird aus Schweinedünndarm-Mucosa durch tryptische Verdauung, Fällung aus 66 % Ethanol bei – 20 °C, Aufnehmen des Präcipitates in 100 mM Phosphat-Puffer, pH 7,0 und abschließende Dialyse gegen denselben Puffer gewonnen.

10 μl einer Probelösung, die so angesetzt ist, daß die Extinktion des Testansates mindestens 10 %, jedoch nicht mehr als 25 % unter der des 100 %-Wertes liegt, werden mit 100 μl einer Verdünnung des intestinalen Disaccharidasen-Komplexes in 0,1 M Maleinat-Puffer, pH 6,25, versetzt und für 10 min bei 37 °C vorinkubiert. Die Verdünnung des Disaccharidasen-Komplexes ist auf eine Aktivität von 0,1 SE/ml einzustellen.

Anschließend wird die saccharolytische Reaktion durch Zugabe von 100 μl einer 0,4 M Lösung von Saccharose (« SERVA 35579 ») in 0,1 M Maleinat-Puffer, pH 6,25 gestartet und nach einer Inkubationsdauer von 20 min bei 37 °C durch die Zugabe von 1 ml Glucose-Dehydrogenase-Reagenz (1 Fläschen Glucose-Dehydrogenase-Mutarotase-Gemisch lyophiliert (« MERCK 14053 ») und 331,7 mg β-Nicotinamid-adenin-dinucleotid (freie Säure, « BOEHRINGER » Reinheitsgrad I) in 250 ml 0,5 M Tris-Puffer, pH 7,6 gelöst) abgestoppt. Zum Nachweis der Glucose wird 30 min bei 37 °C inkubiert und schließlich bei 340 nm gegen einen Reagenzienblank (mit Enzym, jedoch ohne Saccharose) photometriert.

Die Berechnung der Hemmaktivität von Inkubitoren ist dadurch erschwert, daß schon geringfügige Änderungen im Testsystem, beispielsweise ein geringfügig von Bestimmung zu Bestimmung variierender 100 %-Wert, von nicht mehr zu vernachlässigendem Einfluß auf das Testergebnis sind. Man umgeht diese Schwierigkeiten, indem man bei jeder Bestimmung einen Standard mitlaufen läßt; als Standard dient ein Saccharase-Inhibitor der Formel $C_{25}H_{43}O_{18}N$ (Acarbose), welcher eine spezifische Hemmaktivität von 77 700 SIE/g aufweist und bei eingesetzten Mengen von 10 bis 20 ng im Test zu einer Hemmung von oben spezifizierter Größenordnung führt. Bei Kenntnis der Differenz der Extinktionen bei 340 nm von 100 %-Wert und durch Standard gehemmten Ansatz läßt sich aus der Extinktionsdifferenz von 100 %-Wert und durch die Probelösung gehemmten Ansatz unter Berücksichtigung der eingesetzten Menge an Inhibitor in bekannter Weise dessen spezifische Hemmaktivität errechnen, ausgedrückt in Saccharase-Inhibitor-Einheiten pro Gramm (SIE/g).

Saccharase-inhibitorische Aktivität in vitro von Verbindungen der allgemeinen Formel I.

| | SIE/g |
|---|---|
| Vergleich : Substanz der Formel II mit n = 0 und m = 2 (Acarbose) | 77 700 |
| Ia (R : $CH_3$ ; gemäß Bsp. 1) | 79 640 |
| Ib (R : $CH_2CH_3$ ; gemäß Bsp. 2) | 108 310 |
| Ic (R : $CH_2CH_2Cl$ ; gemäß Bsp. 3) | 30 300 |
| Id (R : $CH_2—CH=CH_2$ ; gemäß Bsp. 4) | 93 000 |
| I-α (R : $CH_3$ ; gemäß Bsp. 1b) | 365 200 |
| I-β (R : $CH_3$ ; gemäß Bsp. 1b) | ~ 3 000 |

Die folgenden Beispiele erläutern die Erfindung. In den Beispielen handelt es sich bei allen Prozentangaben um Gewichtsprozente.

Beispiel 1 a (Substanz Ia)

100 g Acarbose (Substanz der Formel II mit n = 0 und m = 2) werden in 1 000 ml abs. Methanol, der 10 % Chlorwasserstoff enthält, gelöst und 4 Stunden bei einer Temperatur von 60 °C gerührt. Danach wird mit festem Natriumcarbonat neutralisiert und der gebildete Niederschlag abfiltriert. Das erhaltene Filtrat wird bei vermindertem Druck zu einem braunen, viskosen Sirup eingeengt. Dieser wird in 500 ml Wasser gelöst und auf eine Säule mit 1 000 ml Ionenaustauscherharz (Amberlite IR 120H®) gegeben. Anschließend wird solange mit Wasser gespült, bis keine nichtbasischen Anteile (Glucose etc.) mehr eluiert werden. Danach wird die Substanz Ia mit 1 %iger wäßriger Ammoniaklösung von der Säule eluiert. Das basische Eluat wird zum Sirup eingeengt, in 300 ml eines Gemisches aus Methanol und Wasser (3 : 1, V/V) aufgenommen und über eine Säule mit 1 000 g Aluminiumoxid (Woelm Neutral) chromatographiert (Laufmittel : Methanol/Wasser 3 : 1 ; V/V). Die substanzahltigen Fraktionen werden konzentriert und das Produkt Ia aus Methanol/Isopropanol (~ 4 : 1 V/V) kristallisiert.

Ausbeute : 21 g Ia

NMR-spektroskopische Charakterisierung der Verbindung Ia (Anomerengemisch).

Ia wurde nach Acetylierung NMR-spektroskopisch untersucht. Dabei konnten folgende chemische Verschiebungen (in ppm) für die Protonen des Aglykons R und des Aminozuckerteils entnommen werden :

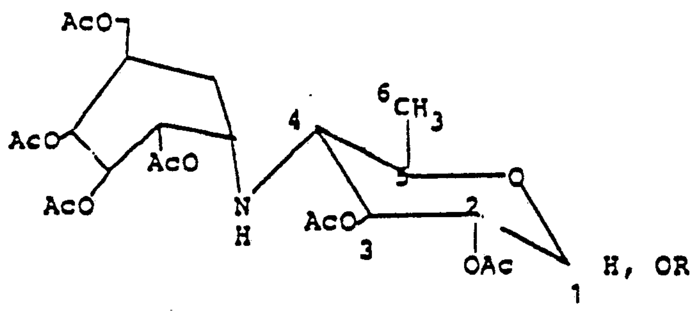

(I ac)

| Substanz | Signal | α-OR | β-OR |
|---|---|---|---|
| I ac | 1-H | 4.80 | 4.31 |
| | 3-H | 5.26 | |
| | 4-H | 2.44 | 2.49 |
| R:$CH_3$ | 5-H | 3.61 | 3.27 |
| | 6-$CH_3$ | 1.25 | 1.31 |
| | R ($CH_3$) | 3.48 | 3.37 |

Die Messungen erfolgten in $CDCl_3$ bei einer Frequenz von 250 MHz.

Beispiel 1 b

Darstellung der reinen α- und β-Anomeren des Methylglykosides Ia.

Durch fraktionielle Kristallisation aus Methanol wurde aus einem Gemisch der Methylglykoside (siehe Beispiel Ia) das β-Anomere soweit abgetrennt, bis die Mutterlauge etwa 80 % des α-Methylglykosides enthielt.

Der nach Einengen der Mutterlauge verbleibende Sirup (17 g) wurde in 200 ml abs. Dimethylformamid gelöst und unter Eiskühlung mit 6 ml 48 %iger HBr-Lösung versetzt. Danach wurde bei Raumtemperatur solange Isopropenylmethylether zugetropft (ca. 100 ml über einen Zeitraum von 2 Stunden), bis die Substanz mit einem Rf-Wert von 0.5 (DC : Kieselgel-Fertigfolie, Merck, Lufmittel Toluol/Ethanol 3 : 1) Hauptprodukt war.

Zur Aufarbeitung wurde der Ansatz mit festem Natriumcarbonat neutralisiert, filtriert und eingeengt. Der Rückstand wurde in 200 ml Chloroform aufgenommen, die organische Phase zweimal mit 100 ml Wasser gewaschen, über Natriumsulfat getrocknet und eingeengt. Rohausbeute 21 g.

Durch präparative Säulenchromatographie über Kieselgel 60 (Merck) mit Toluol/Essigester 2 : 1 (V/V) als Laufmittel wurden 13 g der Di-Isopropyliden-Verbindung V erhalten. Eine analytische Probe wurde zu VI acetyliert, da V nicht kristallisierte.

Zur Darstellung des freien α-Methylglykosides wurde die Verbindung V in 60 %iger wäßriger Essigsäure gelöst und 4 Stunden bei 60 °C gerührt. Die Substanz I-α kristallisierte aus Methanol.

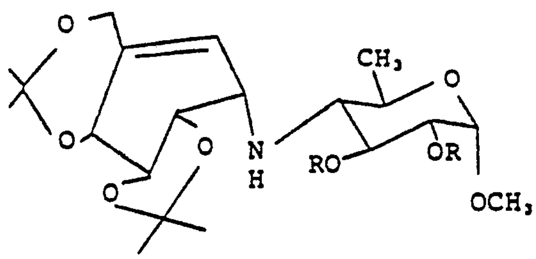

V R = —H

VI R = —$COCH_3$

Physikalische Daten :
I-α Fp 157 °C $[\alpha]_D^{20}$ = + 185.1°
(c = 0.8 $H_2O$)
1-β Fp 208 °C $[\alpha]_D^{20}$ = + 19.8°
(c = 0.5 $H_2O$)
VI Fp 160 °C $[\alpha]_D^{20}$ = + 146.5°
(c = 0.8 $CHCl_3$)

Beispiel 2 (Substanz Ib)

10 g Acarbose werden in 400 ml abs. Ethanol, der 2,5 % Chlorwasserstoff enthält, gelöst und 8 Stunden bei 80 °C gerührt. Die Aufarbeitung erfolgt analog Beispiel 1a, die Umkristallisation aus Methanol.

Ausbeute : 0,7 g Ib

Beispiel 3 (Substanz Ic)

100 g Acarbose werden in 1 000 ml abs. Chlorethanol, der 1,5 % Chlorwasserstoff enthält, gelöst und 12 Stunden bei 60 °C gerührt. Die Aufarbeitung erfolgt wie in Beispiel Ia, die Umkristallisation aus Methanol.

Ausbeute : 19 g Ic

Beispiel 4 (Substanz Id)

10 g Acarbose werden in 400 ml abs. Allylalkohol, der 2 % Chlorwasserstoff enthält, gelöst und 7 Stunden bei 90 °C gerührt. Die Aufarbeitung erfolgt analog zum Beispiel Ia, die Umkristallisation aus Methanol.

Ausbeute : 1,1 g Id

NMR-spektroskopische Charakterisierung der Verbindungen I b-d.

Die Glycoside der allgemeinen Formel I wurden nach Acetylierung NMR-spektroskopisch untersucht. Dabei konnten folgende chemische Verschiebungen (in ppm) für die Protonen des Aglykons R und des Aminozuckerteils entnommen werden :

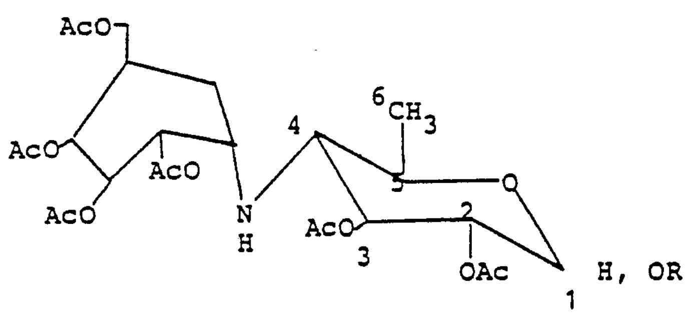

(I ac)

| Substanz | Signal | α-OR | β-OR |
|---|---|---|---|
| Ib ac | 1-H | 4.92 | 4.39 |
| | 2-H | 4.79 | |
| | 3-H | 5.23 | |
| R:$CH_2$-$CH_3$ | 4-H | 2.42 | 2.49 |
| | 5-H | | 3.25 |
| | R: -$CH_2$- | 3.95-3.41 | |
| | $CH_3$ | 1.20 | |
| Ic ac | 1-H | 5.01 | 4.46 |
| | 2-H | 4.78 | |
| | 3-H | 5.28 | |
| R:$CH_2$-$CH_2$-Cl | 4-H | 2.44 | 2.49 |
| | 5-H | | 3.28 |
| | 6-$CH_3$ | 1.24 | 1.30 |
| | R: -$CH_2$-$C_2$-Cl | 4.24-3.60 | |
| Id ac | 1-H | 4.96 | 4.45 |
| | 2-H | 4.82 | |
| | 3-H | 5.30 | |
| R:$CH_2$-CH=$CH_2$ | 4-H | 2.43 | 2.50 |
| | 5-H | 3.65 | 3.26 |
| | 6-$CH_3$ | 1.24 | 1.30 |
| | R: -$CH_2$- | 4.37-3.92 | |
| | -CH= | 5.94-5.76 | |
| | = $CH_2$ | 5.35-5.15 | |

Die Messungen erfolgten in $CDCl_3$ bei einer Frequenz von 250 MHz.

## Patentansprüche

1. Kristalline Verbindungen der allgemeinen Formel

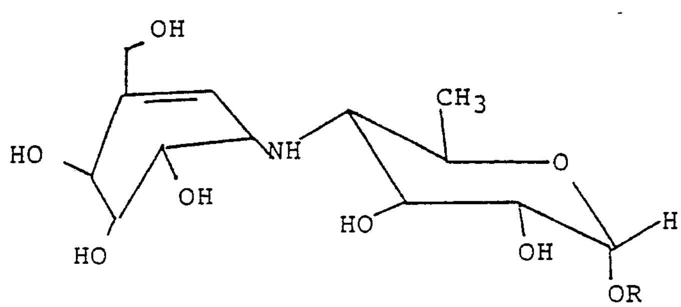

(α-Anomeres)

worin R einen gegebenenfalls durch Alkoxy mit 1 bis 4 Kohlenstoffatomen, Alkylthio mit 1 bis 4 Kohlenstoffatomen, Halogen, oder Cyan, ein- oder mehrfach substituierten Alkyl- oder Alkenylrest mit bis zu 6 C-Atomen darstellt.

2. Verfahren zur Herstellung von kristallinen Verbindungen der allgemeinen Formel

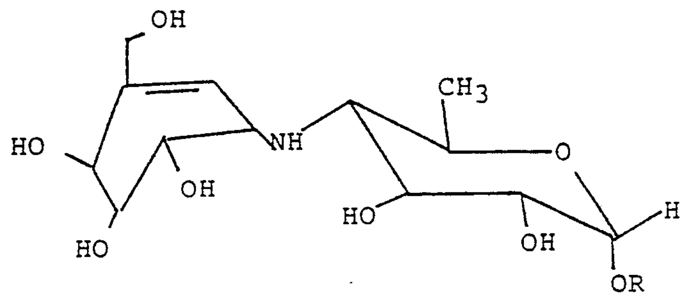

(α-Anomeres)

worin R einen gegebenenfalls durch Alkoxy mit 1 bis 4 Kohlenstoffatomen, Alkylthio mit 1 bis 4 Kohlenstoffatomen, Halogen, oder Cyan, ein- oder mehrfach substituierten Alkyl- oder Alkenylrest mit bis zu 6 C-Atomen darstellt, dadurch gekennzeichnet, daß man Verbindungen der allgemeinen Formel III

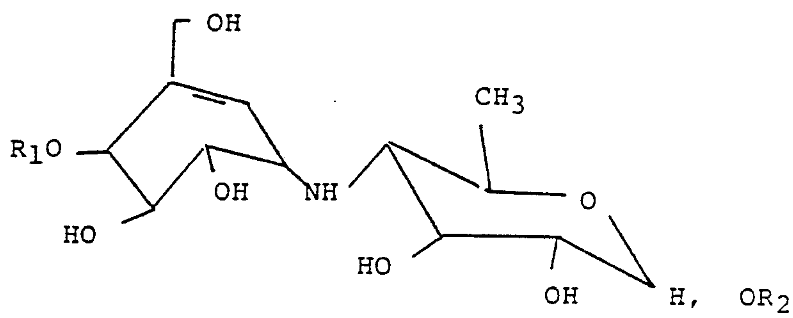

(III)

worin $R_1$ und $R_2$ für gleiche oder verschiedene Mono- oder Oligosaccharidreste stehen, die als $R_1$ glykosidisch und als $R_2$ beliebig über Sauerstoff an das Restmolekül gebunden sind mit einem Alkohol ROH, wobei R die obengenannte Bedeutung hat, in Gegenwart von Protonensäuren HX, worin X den Rest einer starken anorganischen Säure darstellt, umsetzt und das α-Anomere aus dem erhaltenen Gemisch isoliert.

3. Verfahren nach Anspruch 2, dadurch gekennzeichnet, daß die Säurekonzentration etwa 0,5 bis 4 normal ist.

4. Verfahren nach den Ansprüchen 2 oder 3, dadurch gekennzeichnet, daß die Umsetzung bei einer Temperatur von etwa 60 bis etwa 130 °C durchgeführt wird.

5. Verfahren nach den Ansprüchen 2-4, dadurch gekennzeichnet, daß die Umsetzung während etwa 0,5-12 Stunden erfolgt.

6. Verfahren nach den Ansprüchen 2-5, dadurch gekennzeichnet, daß die Isolierung des α-Anomeren durch fraktionierte Kristallisation erfolgt.

7. Kristalline Verbindungen der allgemeinen Formel

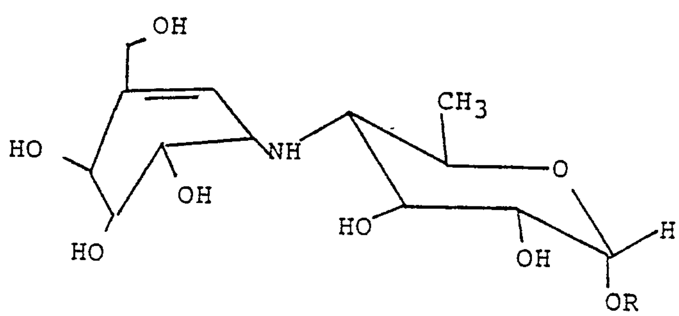

(α-Anomeres)

worin R einen gegebenenfalls durch Alkoxy mit 1 bis 4 Kohlenstoffatomen, Alkylthio mit 1 bis 4 Kohlenstoffatomen, Halogen, oder Cyan, ein- oder mehrfach substituierten Alkyl- oder Alkenylrest mit bis zu 6 C-Atomen darstellt, zur Bekämpfung von Krankheiten.

8. Arzneimittel enthaltend mindestens eine der Verbindungen gemäß Anspruch 1.
9. Verwendung von Verbindungen der allgemeinen Formel

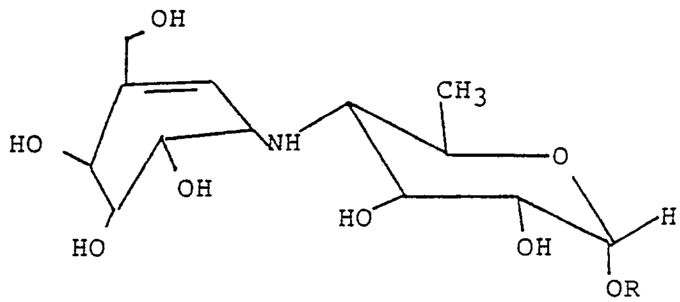

(α-Anomeres)

worin R einen gegebenenfalls durch Alkoxy mit 1 bis 4 Kohlenstoffatomen, Alkylthio mit 1 bis 4 Kohlenstoffatomen, Halogen oder Cyan, ein- oder mehrfach substituierten Alkyl- oder Alkenylrest mit bis zu 6 C-Atomen darstellt,
zur Herstellung von Arzneimitteln.

**Claims**

1. Crystalline compounds of the general formula

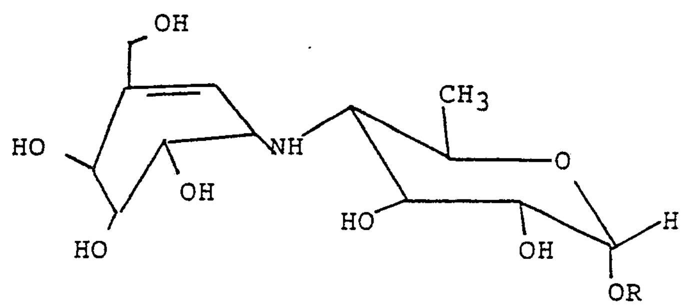

(α-anomer)

wherein R represents an alkyl or alkenyl radical which has up to 6 C atoms and is optionally mono- or polysubstituted by alkoxy with 1 to 4 carbon atoms, alkylthio with 1 to 4 carbon atoms, halogen or cyano.

2. Process for the preparation of crystalline compounds of the general formula

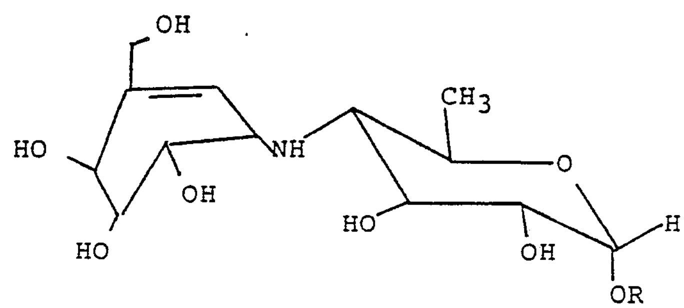

(α-anomer)

wherein R represents an alkyl or alkenyl radical which has up to 6 C atoms and is optionally mono- or polysubstituted by alkoxy with 1 to 4 carbon atoms, alkylthio with 1 to 4 carbon atoms, halogen or cyano, characterised in that compounds of the general formula III

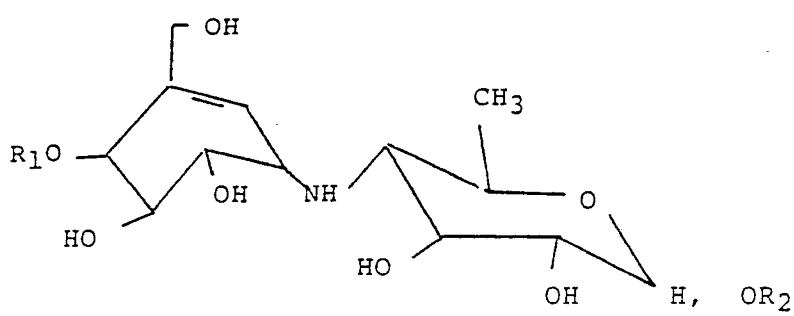

(III)

wherein $R_1$ and $R_2$ represent identical or different mono- or oligosaccharide radicals which, as $R_1$, are bonded glycosidically and, as $R_2$, in any desired manner via oxygen to the remainder of the molecule, are reacted with an alcohol ROH, R having the abovementioned meaning, in the presence of protonic acids HX, X representing the radical of a strong inorganic acid, and the α-anomer is isolated from the mixture obtained.

3. Process according to Claim 2, characterised in that the acid concentration is about 0.5 to 4 normal.

4. Process according to Claims 2 or 3, characterised in that the reaction is carried out at a temperature of about 60 to about 130 °C.

5. Process according to Claims 2-4, characterised in that the reaction is effected during the course of about 0.5-12 hours.

6. Process according to Claims 2-5, characterised in that the isolation of the α-anomer is carried out by fractional crystallisation.

7. Crystalline compounds of the general formula

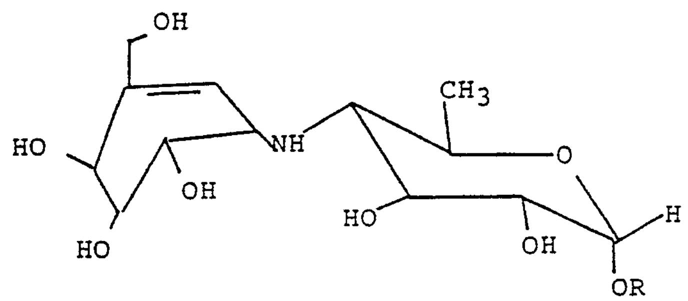

(α-anomer)

wherein R represents an alkyl or alkenyl radical which has up to 6 C atoms and is optionally mono- or polysubstituted by alkoxy with 1 to 4 carbon atoms, alkylthio with 1 to 4 carbon atoms, halogen or cyano, for combating diseases.

8. Medicaments containing at least one of the compounds according to Claim 1.

9. Use of compounds of the general formula

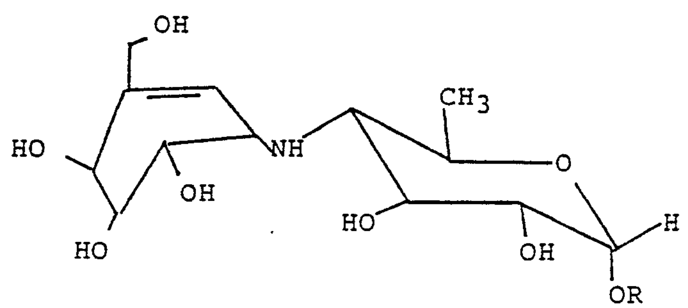

(α-anomer)

wherein R represents an alkyl or alkenyl radical which has up to 6 C atoms and is optionally mono- or polysubstituted by alkoxy with 1 to 4 carbon atoms, alkylthio with 1 to 4 carbon atoms, halogen or cyano, for the preparation of medicaments.

**Revendications**

1. Composés cristallins de formule générale :

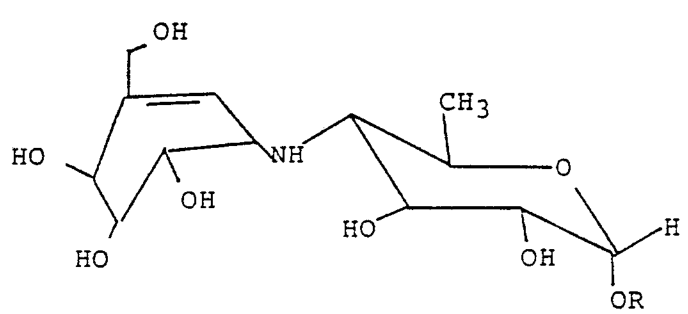

(α-anomères)

dans laquelle R représente un radical alcoyle ou alcényle ayant jusqu'à 6 atomes de carbone, le cas échéant substitué une ou plusieurs fois par un alcoxy ayant 1 à 4 atomes de carbone, alcoylthio ayant 1 à 4 atomes de carbone, un halogène ou un cyano.

2. Procédé de fabrication de composés cristallins de formule générale :

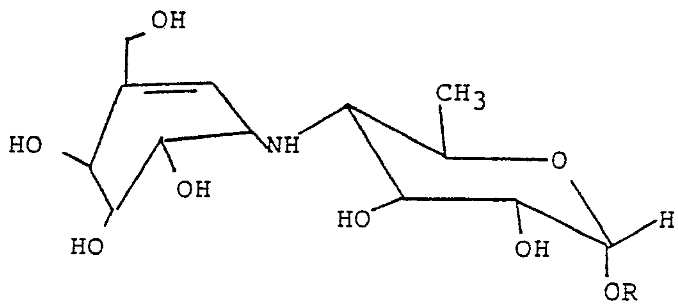

(α-anomères)

dans laquelle R représente un radical alcoyle ou alcényle ayant jusqu'à 6 atomes de carbone, éventuellement substitué une ou plusieurs fois par un alcoxy ayant 1 à 4 atomes de carbone, un alcoylthio ayant 1 à 4 atomes de carbone, un halogène ou un cyano,
caractérisé en ce qu'on fait réagir des composés de formule générale III

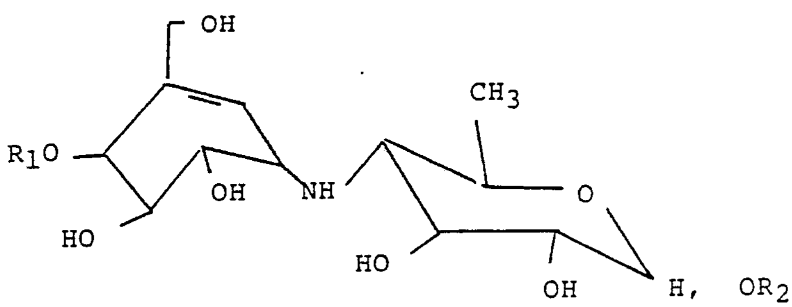

(III)

dans laquelle $R_1$ et $R_2$ représentent des radicaux de mono- ou oligosaccharide, identiques ou différents qui, en tant que $R_1$, sont reliés glycosidiquement et, en tant que $R_2$, de manière quelconque par de l'oxygène à la molécule restante, avec un alcool ROH où R a la signification indiquée plus haut, en présence d'acides protoniques HX où X représente le radical d'un acide minéral fort, et en ce qu'on isole l'anomère α à partir du mélange obtenu.

3. Procédé selon la revendication 2, caractérisé en ce que la concentration d'acide est environ 0,5 à 4 fois normale.

4. Procédé selon la revendication 2 ou 3, caractérisé en ce qu'on exécute la réaction à une température d'environ 60 à environ 130 °C.

5. Procédé selon les revendications 2 à 4, caractérisé en ce que la réaction a lieu pendant environ 0,5 à 12 heures.

6. Procédé selon les revendications 2 à 5, caractérisé en ce que l'isolation de l'anomère se fait par cristallisation fractionnée.

7. Composés cristallins de formule générale :

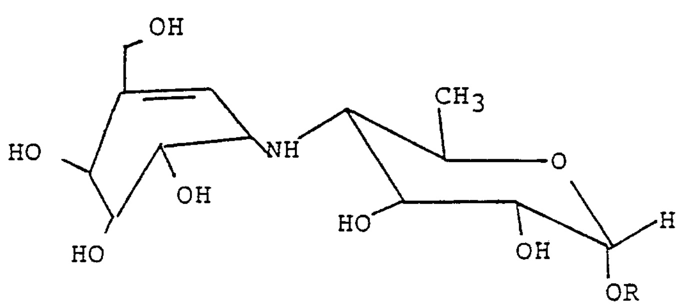

(α-anomères)

dans laquelle R représente un radical alcoyle ou alcényle ayant jusqu'à 6 atomes de carbone, éventuellement mono- ou polysubstitué par un alcoxy ayant 1 à 4 atomes de carbone, un alcoylthio ayant 1 à 4 atomes de carbone, un halogène ou un cyano, pour combattre des maladies.

8. Médicaments contenant au moins un des composés selon la revendication 1.
9. Utilisation de composés de formule générale :

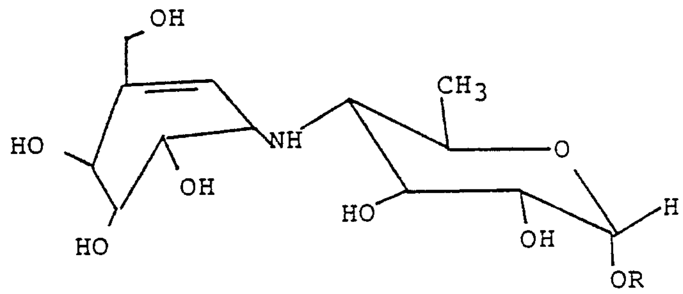

(α-anomères)

dans laquelle R représente un radical alcoyle ou alcényle ayant jusqu'à 6 atomes de carbone, éventuellement mono- ou polysubstitué par un alcoxy ayant 1 à 4 atomes de carbone, un alcoylthio ayant 1 à 4 atomes de carbone, un halogène ou un cyano, en vue de la fabrication de médicaments.